# EUROPÄISCHE PATENTSCHRIFT

(11) **EP 1 846 367 B1**
(45) Veröffentlichungstag und Bekanntmachung des Hinweises auf die Patenterteilung: **09.03.2016**
(21) Anmeldenummer: 05803251.7
(22) Anmeldetag: 14.10.2005
(51) Int. Cl.: C07C 265/14, C07C 263/18

(54) **LAGERSTABILE ALIPHATISCHE, CYCLOALIPHATISCHE ODER (CYCLO)ALIPHATISCHE DIISOCYANATE**
ALIPHATIC, CYCLOALIPHATIC OR (CYCLO)ALIPHATIC DIISOCYANATES THAT ARE STABLE IN STORAGE
DIISOCYANATES ALIPHATIQUES, CYCLOALIPHATIQUES OU (CYCLO)ALIPHATIQUES STABLES AU STOCKAGE

(30) Priorität: 02.12.2004 DE 102004058173
(43) Veröffentlichungstag der Anmeldung: 24.10.2007
(73) Patentinhaber: Evonik Degussa GmbH, 45128 Essen (DE)
(72) Erfinder: HOPPE, Dirk, 48301 Nottuln (DE); LOMÖLDER, Rainer, 48153 Münster (DE); KOHLSTRUK, Stephan, 48249 Dülmen (DE); MICHALCZAK, Hans-Werner, 44651 Herne (DE)
(86) Internationale Anmeldenummer: PCT/EP2005/055271
(87) Internationale Veröffentlichungsnummer: WO 2006/058807

(56) Entgegenhaltungen:
- EP-A- 0 531 803
- EP-A- 0 643 042
- EP-A1- 1 273 603
- EP-A1- 1 362 873
- DE-A1- 4 331 085
- DATABASE WPI Section Ch, Week 199732 Derwent Publications Ltd., London, GB; Class E14, AN 1997-347441 XP002362014 & JP 09 143141 A (MITSUBISHI GAS CHEM CO INC) 3. Juni 1997 (1997-06-03)
- DATABASE WPI Section Ch, Week 199333 Derwent Publications Ltd., London, GB; Class E14, AN 1993-261623 XP002362015 & JP 05 178811 A (MITSUI TOATSU CHEM INC) 20. Juli 1993 (1993-07-20)

## Beschreibung

Die Erfindung betrifft die Verwendung von Sauerstoff zur Stabilisierung eines aliphatischen, cycloaliphatischen oder (cyclo)aliphatischen Diisocyanats sowie Mischungen davon.

Organische Polyisocyanate, wie z. B. aromatische, cycloaliphatische, (cyclo)aliphatische und aliphatische Di- und höherwertige Polyisocyanate werden technisch üblicherweise durch Umsetzung der entsprechenden Amine mit Phosgen (Phosgenierung) und die Spaltung der dabei entstehenden Polycarbaminsäurechloriden hergestellt.

Problematisch bei dieser Verfahrensweise sind der hohe Umsatz von Chlor über Phosgen und Carbaminsäurechlorid zu Chlorwasserstoff, die Toxizität des Phosgens sowie die damit verbundenen kostspieligen Sicherheitsvorkehrungen, die Korrosivität der Reaktionsmischung, die Labilität der üblicherweise eingesetzten Lösungsmittel und die Bildung von chlorhaltigen und chlorfreien Nebenprodukten, die physikalische Eigenschaften, wie z. B. die Farbe, die Viskosität und den Dampfdruck, sowie chemische Eigenschaften, wie z. B. die Reaktivität, die Lagerbeständigkeit u. a. der Polyisocyanate sowie die mechanischen Eigenschaften der aus solchen Polyisocyanaten hergestellten Polyisocyanat-Polyadditionsprodukten.

Alternativ können Diisocyanate, wie 1,6-Hexamethylendiisocyanat (HDI), 2,2,4-Trimethylhexamethylendiisocyanat (1,6) und dessen 2,4,4-Trimethylisomeres (TMDI) sowie 1-Isocyanato-3-isocyanatomethyl-3,5,5,-trimethylcyclohexan (Isophorondiisocyanat, IPDI) gemäß der EP 126 299 (USP 4 596 678), EP 126 300 (USP 4 596 679), EP 355 443 (USP 5 087 739) und EP 568 782 (USP 5 360 931) in Kreislaufverfahren hergestellt werden durch Umsetzung der entsprechenden Diamine mit Harnstoff und Alkoholen und gegebenenfalls N-unsubstituierten Carbaminsäureestern, Dialkylcarbonaten und anderen aus dem Reaktionsprozess zurückgeführten Nebenprodukten zu Bis-carbaminsäureestern und deren thermische Spaltung in die entsprechenden Diisocyanate und Alkohole.

Des Weiteren können Diisocyanate, wie zum Beispiel IPDI, kernhydriertes MDI (H₁₂MDI) und kernhydriertes TDI (H₆TDI), unter Verwendung von Dimethylcarbonat nach einer ähnlichen Technologie wie die oben beschriebenen Diisocyanate, ebenfalls unter Vermeidung von Chlor als Rohstoff, hergestellt werden (EP 976 723).

Die Lagerbeständigkeit, die Reaktivität und die Farbe der Diisocyanate und der daraus hergestellten Produkte hängen von Nebenprodukten teilweise unbekannter Struktur ab, die in den Diisocyanaten enthalten sind. Art und Menge dieser Nebenprodukte hängen vom Herstellungsverfahren ab. Es hat sich herausgestellt, dass insbesondere die bei der Phosgenierung auftretenden chlorhaltigen Nebenkomponenten die Lagerbeständigkeit, die Reaktivität und Farbe der aus den Diisocyanaten hergestellten Produkte beeinflussen.

Nach Angaben der USP 3 330 849 können z. B. organische Polyisocyanate durch den Zusatz von Sulfonylisocyanaten gegen eine Verfärbung und Niederschlagsbildung stabilisiert werden. Durch den Zusatz von Metallnaphthenaten, wie z. B. Cadmium-, Kobalt-, Kupfer-, Blei-, Mangan- oder Zinknaphthenat, kann der hydrolysierbare Chlorgehalt von Isocyanaten gemäß USP 3 373 182 reduziert werden. Die USP 3 384 653 und USP 3 449 256 beschreiben die Verbesserung der Lagerbeständigkeit von 4,4-Diphenylmethan-diisocyanat durch eine Behandlung bei 160 bis 250 °C mit Trialkylphosphaten. Der Gehalt an hydrolysierbaren Chlorverbindungen kann gemäß USP 3 458 558 bei organischen Isocyanaten auch mit Kupfer, Silber, Nickel, Eisen und Zink bei Temperaturen über 100 °C erniedrigt werden. Nach Angaben der USP 3 479 393 stabilisieren Trialkylaminborane Isocyanate gegen eine Verfärbung. Gemäß USP 3 535 359 eignen sich Ortho-carbonsäureester zur Stabilisierung von organischen Isocyanaten gegen eine Viskositätserhöhung. Diphenylmethandiisocyanat enthaltende Polyisocyanatmischungen können nach Angaben der USP 3 585 229 durch Zusatz von Diphenyldecylphosphit entfärbt werden. Gegen eine Zersetzung können organische Polyisocyanate gemäß USP 3 692 813 mit Hilfe von Oxycarbonylisocyanaten mit mindestens einer Gruppe der Formel -O-CO-NCO stabilisiert werden. Zur Stabilisierung von organischen Polyisocyanaten gegen eine Verfärbung kann nach Angaben der USP 3 715 381 2,6-Di-tert.-butyl-p-kresol eingesetzt werden. Diphenylmethan-diisocyanate können gemäß USP 3 970 680 auch durch Zugabe von tertiären Aminen stabilisiert werden. Zur Reinigung von organischen Isocyanaten können diese gemäß USP 4 065 362 bei Temperaturen über 100 °C mit einem Metallsalz des Mercaptobenzthiazol, einem Metallsalz von alkylsubstituierten Dithiocarbaminsäuren, einem alkylsubstituierten Phenol, einem Thiobisphenol oder einem Triarylphosphit behandelt werden. Nach Angaben der USP 3 247 236 können durch Umsetzung von Diaminen mit Phosgen hergestellte und durch Destillation gereinigte Diisocyanate durch Zusatz von Kohlendioxid oder Schwefeldioxid stabilisiert werden. Nachteilig an diesem Verfahren ist die gute Löslichkeit des Schwefeldioxids im Polyisocyanat und die Ausbildung von Verfärbungen während der Lagerung. Die genannten Patentpublikationen vermitteln keine Lehre hinsichtlich der Stabilisierung von nach phosgenfreien Verfahren, vorzugsweise von durch thermische Spaltung von organischen Polycarbaminsäureestern, hergestellten organischen Polyisocyanaten.

Ein Nachteil der nach den oben beschriebenen phosgenfreien Verfahren hergestellten aliphatischen, cycloaliphatischen oder (cyclo)aliphatische Diisocyanate ist ihre Tendenz, im Laufe der Lagerung lineare Polymere mit der folgenden (Nylon-1) Struktur 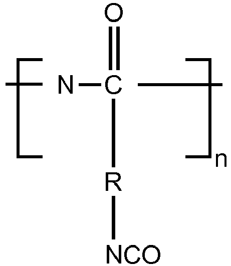 zu bilden. Im Fall des Hexamethylendiisocyanats (HDI) können diese Polymere zu einer Vergelung führen, während bei aus Trimethylhexamethylendiisocyanat (TMDI) hergestellten Produkten nicht gewünschte Trübung beobachtet werden kann.

DE 43 31 085 beschreibt die Stabilisierung nach phosgenfreien Verfahren hergestellter aliphatischer, cycloaliphatischer oder (cyclo)aliphatischer Diisocyanate durch Verwendung von Kohlendioxid als Stabilisator.

EP 643 042 beschreibt die Stabilisierung nach phosgenfreien Verfahren hergestellter aliphatischer, cycloaliphatischer oder (cyclo)aliphatischer Diisocyanate. Als Stabilisator werden Verbindungen, die als Antioxidanz und/oder Radikalfänger dienen (primäre Stabilisatoren), die als Peroxidspalter und/oder Reduktionsmittel wirken (sekundäre Stabilisatoren), und saure Verbindungen (saure Stabilisatoren) oder Gemische der einzelnen Stabilisatorengruppen beschrieben.

Primäre Stabilisatoren im Sinne der EP 643 042 allein sind als Stabilisatoren nicht geeignet, weil sie die Oligomerisierung aliphatischer, cycloaliphatischer oder (cyclo)aliphatischer, nach einem phosgenfreien Verfahren hergestellten Diisocyanate fördern, wie diesseits festgestellt wurde.

Aufgabe der Erfindung war es deshalb, aliphatische, cycloaliphatische und (cyclo)aliphatische, nach einem phosgenfreien Verfahren hergestellte Diisocyanate bezüglich Oligomerisierung und Farbe zu stabilisieren.

Überraschenderweise konnte diese Aufgabe durch Behandlung der aliphatischen, cycloaliphatischen und (cyclo)aliphatischen Diisocyanate mit trockener Luft und/oder trockener synthetischer Luft und/oder trockenem Sauerstoff gelöst werden, die während oder bevorzugt direkt im Anschluss an die Synthese durch die aliphatischen, cycloaliphatischen oder (cyclo)aliphatische Diisocyanate geperlt wurde. Gegebenenfalls können zusätzlich weitere, an sich bekannte Stabilisatoren eingesetzt werden.

Gegenstand der Erfindung ist die Verwendung von Sauerstoff zur Stabilisierung eines aliphatischen, cycloaliphatischen oder (cyclo)aliphatischen Diisocyanats sowie Mischungen davon, hergestellt durch ein phosgenfreies Verfahren.
Die Einbringung des Sauerstoffs in das Diisocyanat bzw. in die Diisocyanatmischung erfolgt vorzugsweise im Anschluß an die Diisocyanatsynthese. Grundsätzlich ist es auch möglich, die phosgenfreie Synthese der aliphatischen, cycloaliphatischen oder (cyclo)aliphatische Diisocyanate bereits in Gegenwart von Sauerstoff durchzuführen.

Die lagerstabilen, aliphatischen, cycloaliphatischen oder (cyclo)aliphatischen Diisocyanate enthalten bei 25 °C und Normaldruck 1 bis 300 ppm (0,0007 bis 0,21 Mol-%) Sauerstoff, bevorzugt 3 bis 200 ppm (0,002 bis 0,14 Mol-%) Sauerstoff, besonders bevorzugt 5 bis 100 ppm (0,004 bis 0,07 Mol-%) Sauerstoff, wobei der Sauerstoff durch reinen trockenen Sauerstoff und/oder trockene Luft und/oder trockene synthetische Luft eingebracht werden kann. Außerdem ist es möglich, dass in Sauerstoff weitere inerte Gase, wie Stickstoff und/oder Edelgase, enthalten sind.

Bewährt hat sich auch, das Diisocyanat bzw. die Diisocyanatmischung zusätzlich im Lagerbehälter (z. B. Lagertank oder Fass) mit sauerstoffhaltiger Atmosphäre zu überlagern.

Die erfindungsgemäßen Diisocyanatzusammensetzungen können beliebige aliphatische, cycloaliphatische und (cyclo)aliphatische Diisocyanate enthalten, mit der Maßgabe, dass diese nach geeigneten Verfahren in Abwesenheit von Phosgen hergestellt werden. Vorzüglich bewährt haben sich und daher vorzugsweise Verwendung finden aliphatische, cycloaliphatische und (cyclo)aliphatische Diisocyanate, die durch thermische Spaltung von aliphatischen, cycloaliphatischen und (cyclo)aliphatischen Dicarbaminsäureestern erhältlich sind. Geeignete aliphatische Diisocyanate besitzen vorteilhafterweise 3 bis 16 Kohlenstoffatome, vorzugsweise 4 bis 12 Kohlenstoffatome, im linearen oder verzweigten Alkylenrest und geeignete cycloaliphatische oder (cyclo)aliphatische Diisocyanate vorteilhafterweise 4 bis 18 Kohlenstoffatome, vorzugsweise 6 bis 15 Kohlenstoffatome, im Cycloalkylenrest. Beispielhaft genannt seien: 1,4-Diisocyanotobutan, 2-Ethyl-1,4-diisocyanato-butan, 1,5-Diisocyanto-pentan, 2,2-Dimethyl-1,5-diisocyanato-pentan, 2-Methyl-1,5-diisocyanato-pentan (MPDI), 2-Ethyl-2-propyl-1,5-diisocyanato-pentan, 2-Ethyl-2-butyl-1,5-diisocyanatopentan, 2-Alkoxy-1,5-diisocyanato-pentan, 1,6-Hexamethylendiisocyanat (HDI), 2,4,4- bzw. 2,2,4-Trimethyl-1,6-hexamethylen-diisocyanat (TMDI), 1,7-Diisocyanato-heptan, 1,8-Diisocyanato-octan, 1,10-Diisocyanato-decan, 1,12-Diisocyanato-dodecan, 4,4'-Diisocyanato-dicyclohexylmethan, 2,4'-Diisocyanato-dicyclohexylmethan sowie Gemische der isomeren Diisocyanatodicyclohexylmethane (H12MDI), 1,3-Diisocyanato-cyclohexan sowie Isomerengemische von Diisocyanato-cyclohexanen und 1-Isocyanato-3-isocyanatomethyl-3,5,5-trimethylcyclohexan (IPDI).

Vorzugsweise werden als aliphatische, cycloaliphatische und (cyclo)aliphatische Diisocyanate 1-Isocyanato-3-isocyanatomethyl-3,5,5-trimethylcyclohexan, 2,4,4- bzw. 2,2,4-Trimethyl-1,6-hexamethylen-diisocyanat, 4,4'-Diisocyanato-dicyclohexylmethan, 2,4'-Diisocyanato-dicyclohexylmethan sowie Gemische der isomeren Diisocyanato-dicyclohexylmethane sowie 1,6-Hexamethylen-diisocyanat eingesetzt.

Besonders bevorzugt sind IPDI, H₁₂MDI, TMDI, HDI und/oder MPDI enthalten.

Wie bereits dargelegt, werden die aliphatischen, cycloaliphatischen und (cyclo)aliphatischen Diisocyanate vorzugsweise durch thermische Spaltung der entsprechenden Dicarbaminsäureester hergestellt. Diese Spaltung kann beispielsweise bei Temperaturen von 150 bis 300 °C, vorzugsweise 180 bis 250 °C, und Drücken von 0,001 bis 2 bar, vorzugsweise von 1 bis 200 mbar, in Abwesenheit oder vorzugsweise Gegenwart von Katalysatoren in geeigneten Spaltreaktoren, wie z. B. Dünnschichtverdampfern, Fallfilmverdampfern oder Heizkerzenverdampfern nach der EP 524 554 durchgeführt werden. Die bei der Spaltung entstehenden Diisocyanate und Alkohole können beispielsweise durch fraktionierte Kondensation oder vorzugsweise durch Rektifikation getrennt und die Diisocyanate z. B. durch Destillation zusätzlich gereinigt werden.

Die erfindungsgemäß stabilisierten, nach einem phosgenfreien Verfahren hergestellten aliphatischen, cycloaliphatischen und (cyclo)aliphatischen Diisocyanate können durch trockene Luft oder Sauerstoff allein stabilisiert werden. Zusätzlich können andere stabilisierend wirkende Verbindungen verwendet werden.

Als zusätzliche Stabilisatoren gegen Verfärbung sind beispielsweise primäre Stabilisatoren geeignet, die üblicherweise als Antioxidantien und/oder als Radikalfänger wirksam sind. Primäre Stabilisatoren im Sinne dieser Erfindung sind beispielsweise phenolische Antioxidatien, die mindestens eine sterisch gehinderte phenolische Gruppierung enthalten. Beispiele für diese phenolischen Antioxidantien sind: 2,6-Di-tert.-butyl-4-methylphenol, 2,4,6-Tri-tert.-butylphenol, 2,2'-Methylen-bis-(4-methyl-6-tert.-butylphenol), 2,2'-Thio-bis-(4-methyl-6-t-butylphenol), 4,4'-Thio-bis-(3-methyl-6-t-butylphenol), 4,4'-Butyliden-bis-(3-methyl-6-tert.-butylphenol), 4,4'-Methyliden-bis-(2,6-di-tert.-butylphenol), 2,2'-Methyliden-bis-[4-methyl-6-(1-methylcyclohexyl)phenol], Tetrakis-[methylen-3-(3,5-di-tert.-butyl-4-hydroxyphenyl)-propionat]methan, 1,3,5-Trimethyl-2,4,6-tris(3,5-di-tert.-butyl-4-hydroxybenzyl)benzol, N,N'-Hexamethylen-bis-(3,5-di-tert.-butyl-4-hydroxyhydrozimtsäureamid), Octadecyl-3-(3,5-di-tert.-butyl-4-hydroxyphenyl)-propionat, 1,3,5-Tris-(3,5-di-tert.-butyl-4-hydroxybenzyl)-isocyanurat, 1,1,3-Tris-(5-tert.-butyl-4-hxdroxy-2-methylphenyl)butan, 1,3,5-Tris-(3,5-di-tert.-butyl-4-hydroxybenzyl)mesitylen; Ethylenglycol-bis[3,3-bis(3'-tert.-butyl-4'-hydroxyphenyl)butyrat], 2,2'-Thiodiethyl-bis-3-(3,5-di-tert.-butyl-4-hydroxyphenyl)propionat, 2,2'-Methylen-bis-(4-methyl-6-cyclohexylphenol), 1,6-Hexandiol-bis(3,5-di-tert.-butyl-4-hydroxyphenyl)propionat, 2,4-Bis-(n-octylthio)-6-(4-hydroxy-3,5-di-tert.-butylanilino)-1,3,5-triazin, 3,5-Di-tert.-butyl-4-hydroxybenzyl-phosphon-säurediethylester und Triethylenglykol-bis-3-(tert.-butyl-4-hydroxy-5-methyl-phenyl)propionat.

Bevorzugt eingesetzt werden Octadecyl-3-(3,5-di-ter.t-butyl-4-hydroxyphenyl)-propionat, 2,2'-Methylen-bis-(4-methyl-6-cyclohexylphenol), 2,2'-Methylen-bis-(4-methyl-6-tert.-butylphenol), Triethylenglykol-bis-3-(tert.-butyl-4-hydroxy-5-methylphenyl)propionat, Tetrakis-[methylen-3-(3,5-di-tert.-butyl-4-hydroxyphenyl)-propionat]methan und 2,6-Di-tert.-butyl-4-methylphenol.

Die primären Stabilisatoren finden in einer Menge von 1 bis 300 ppm, vorzugsweise in einer Menge von 1 bis 200 ppm, bezogen auf das Gewicht der Diisocyanatzusammensetzung, Verwendung.

Des Weiteren können als Stabilisatoren sekundäre Stabilisatoren, die üblicherweise als Peroxidspalter und/oder Reduktionsmittel wirksam sind, eingesetzt werden. Geeignete sekundäre Stabilisatoren sind z. B. phosphorhaltige Verbindungen, vorzugsweise Triester der phosphorigen Säure, wie zum Beispiel Trialkyl- und Triarylphosphite und Thioether.

Beispiele für die Ester der phosphorigen Säure sind Distearylpentaerythritdiphosphit, Tris-(nonylphenyl)-phosphit, Tris-(2,4-di-tert.-butylphenyl)phosphit, Neopentylglykoltriethylenglykoldiphosphit, Diisodecylpentaerythritdiphosphit, Tristearylphosphit, Trilaurylphosphit und insbesondere Triphenylphosphit.

Beispiele für die Thioether sind 2-Methyl-1-propenyl-tert.-dodecylthioether, Cyclohexylidenmethyl-n-dodecylthioether, 3-Cyclohexen-(1)-ylidenmethyl-n-ocatadecylthioether, 3-Cyclohexen-(1)-ylidenmethyl-n-dodecylthioether, 3-Cyclohexen-(1)-ylidenmethyl-n-octylthioether, 3-Cyclohexen-(1)-ylidenmethyl-n-cyclohexylthioether, 3-Cyclohexen-(1)-ylidenmethyl-p-tolylthioether und 3-Cyclohexen-(1)-ylidenmethyl-benzylylthioether.
Die sekundären Stabilisatoren finden in einer Menge von 1 bis 300 ppm, vorzugsweise in einer Menge von 1 bis 200 ppm, bezogen auf das Gewicht der Diisocyanatzusammensetzung, Verwendung.

In einer weiteren bevorzugten Ausführungsform der Erfindung sind unter Normalbedingungen 1 bis 200 ppm (0,0007 bis 0,14 Mol-%) Sauerstoff und 1 bis 200 ppm (0,0002 bis 0,02 Mol-%) eines primären Stabilisators a) zur Farbreduzierung enthalten. In einer weiteren bevorzugten Ausführungsform der Erfindung sind unter Normalbedingungen 5 bis 100 ppm (0,0007 bis 0,07 Mol-%) Sauerstoff und 1 bis 150 ppm (0,0002 bis 0,015 Mol-%) 2,6-Di-tert.-butyl-4-methylphenol enthalten.

Ferner können zusätzlich zum Sauerstoff Zusammensetzungen eingesetzt werden, die durch partielle Umsetzung von Sauerstoff mit primären und/oder sekundären Stabilisatoren erhalten werden. Weitere bevorzugte Ausführungsformen der Erfindung sind: mit Sauerstoff und/oder Luft stabilisiertes TMDI; mit Sauerstoff und/oder mit Luft und einem Trialkylphosphit, insbesondere aus Tributyl- und/oder Triphenylphosphit stabilisiertes TMDI; mit Sauerstoff und/oder mit Luft stabilisiertes H₁₂MDI; mit Sauerstoff und/oder mit Luft und 2,6-Di-tert.-butyl-4-methylphenol stabilisiertes H₁₂MDI, mit Sauerstoff und/oder Luft stabilisiertes IPDI, mit Sauerstoff und/oder mit Luft stabilisiertes IPDI und 2,6-Di-tert.-butyl-4-methylphenol.

Bevorzugt wird die trockene Luft und/oder der trockene Sauerstoff mit einer Düse, einer Fritte oder durch Überlagerung einer turbulenten Strömung in das Diisocyanat eingebracht, wobei besonders bevorzugt die Luft und/oder der Sauerstoff im Anschluss an den Reindestillationsschritt der Diisocyanatsynthese eingebracht wird. Die Luft und/oder der Sauerstoff werden bei Temperaturen von -20 °C bis 200 °C, bei einem Druck von 5 mbar bis 15 bar, mit einem Volumensstrom von 0,001 bis 100000 Liter pro Stunde eingebracht.

Außerdem können weitere inerte Gase, wie z. B. Stickstoff oder Edelgase, wie z. B. Argon, zusätzlich enthalten sein.

Die folgenden Beispiele sollen die Erfindung näher erklären.

### Beispiele

Die Ausprägung der bei einigen Folgeprodukten des nach einem phosgenfreien Verfahren hergestellten 2,2,4-(2,4,4)-Trimethylhexamethylendiisocyanat (TMDI) beobachteten Trübungen korrelieren ausgezeichnet mit der Trübungsneigung von 10%igen Lösungen des TMDI in Acetonitril oder Propylencarbonat, sodass diese Lösungen als Schnelltest zur Beurteilung der Stabilität des TMDI herangezogen werden können. Die Trübungsneigung der Folgeprodukte nimmt mit zunehmender Lagerung des TMDI zu. Die Trübung der 10%igen Lösungen von TMDI in Acetonitril oder Propylenglykol kann mit Hilfe einer Transmissionsmessung zwischen 350 und 900 nm in Anlehnung an die DIN EN 1557 (LICO 200 der Fa. Dr. Lange) quantitativ bestimmt werden.

### Vergleichsbeispiel 1

100 g eines nicht stabilisierten, nach einem phosgenfreien Verfahren hergestellten 2,2,4-(2,4,4)-Trimethylhexamethylendiisocyanats (TMDI) wurden direkt nach der Produktion in 900 g Propylencarbonat gelöst. Das Gemisch war optisch klar und wies bei einer Wellenlänge von 550 nm eine Transmission von 100 % auf. Weitere Proben des TMDI wurden bei Raumtemperatur gelagert und nach vier, sechs, acht und zehn Monaten im Verhältnis 1 : 9 in Propylenglykol gelöst. Die Ergebnisse der Transmissionsmessung sind in Tabelle 1 zusammengefasst. Die Messung der Transmission erfolgte jeweils eine Stunde nach Lösen des TMDI in Propylenglykol.

### Beispiel 1

Direkt nach Herstellung wurde bei einer Temperatur von 25 °C trockene synthetische Luft mit einem Volumenstrom von 5 l pro Stunde über eine Fritte durch 100 g eines nach einem phosgenfreien Verfahren hergestellten 2,2,4-(2,4,4)-Trimethylhexamethylendiisocyanats (TMDI) geleitet. Die Sättigungskonzentration des Sauerstoffs im TMDI betrug unter diesen Bedingungen 0,025 Mol-%. Das so stabilisierte TMDI wurde in Propylenglykol gelöst bzw. entsprechend des Vergleichsbeispiels gelagert und nach den in der folgenden Tabelle angegebenen Zeiten in Propylenglykol gelöst. Die Messung der Transmission erfolgte jeweils eine Stunde nach Lösen des TMDI in Propylenglykol. Die folgende Tabelle fasst die Ergebnisse des erfindungsgemäßen Beispiels und des Vergleichsbeispiels zusammen:

| Lagerdauer [Monate] | Transmission [%] | |
|---|---|---|
| | Vergleichsbeispiel 1 | Beispiel 1 |
| 0 | 100 | 100 |
| 4 | 75 | 95 |
| 6 | 70 | 88 |
| 8 | 60 | 84 |
| 10 | 50 | 75 |

### Vergleichsbeispiel 2

100 g eines mit 100 ppm 2,6-Di-tert.-butyl-4-methylphenol stabilisierten, nach einem phosgenfreien Verfahren hergestellten 2,2,4-(2,4,4)-Trimethylhexamethylendiisocyanats (TMDI) wurden direkt nach der Produktion in 900 g Propylencarbonat gelöst. Das Gemisch war optisch klar und wies bei einer Wellenlänge von 550 nm eine Transmission von 100 % auf Weitere Proben des mit 100 ppm 2,6-Di-tert.-butyl-4-methylphenol stabilisierten TMDI wurden bei Raumtemperatur gelagert und nach vier, sechs, acht und zehn Monaten im Verhältnis 19 in Propylenglykol gelöst. Die Ergebnisse der Transmissionsmessung sind in Tabelle 1 zusammengefasst. Die Messung der Transmission erfolgte jeweils eine Stunde nach Lösen des TMDI in Propylenglykol.

### Beispiel 2

Direkt nach Herstellung wurde bei einer Temperatur von 25 °C trockene synthetische Luft mit einem Volumenstrom von 5l pro Stunde über eine Fritte durch 100 g eines mit 100 ppm 2,6-Di-tert.-butyl-4-methylphenol stabilisierten, nach einem phosgenfreien Verfahren hergestellten 2,2,4-(2,4,4)-Trimethylhexamethylendiisocyanats (TMDI) geleitet. Die Sättigungskonzentration des Sauerstoffs im TMDI betrug unter diesen Bedingungen 0,025 Mol-%. Das so stabilisierte TMDI wurde in Propylenglykol gelöst bzw. entsprechend des Vergleichsbeispiels gelagert und nach den in der folgenden Tabelle angegebenen Zeiten in Propylenglykol gelöst. Die Messung der Transmission erfolgte jeweils eine Stunde nach Lösen des TMDI in Propylenglykol. Die folgende Tabelle fast die Ergebnisse des erfindungsgemäßen Beispiels und des Vergleichsbeispiels zusammen:

| Lagerdauer [Monate] | Transmission [%] | |
|---|---|---|
| | Vergleichsbeispiel 2 | Beispiel 2 |
| 0 | 100 | 100 |
| 4 | 40 | 80 |
| 6 | nicht messbar | 70 |
| 8 | nicht messbar | 60 |

## Patentansprüche

1. Verwendung von Sauerstoff zur Stabilisierung eines aliphatischen, cycloaliphatischen oder (cyclo)aliphatischen Diisocyanats sowie Mischungen davon, hergestellt durch ein phosgenfreies Verfahren.

2. Verwendung nach Anspruch 1,
**dadurch gekennzeichnet,**
**dass** der Sauerstoff bei 25 °C und Normaldruck in einer Konzentration von 1 bis 300 ppm (0,0007 bis 0,21 Mol-%) vorliegt.

3. Verwendung nach Anspruch 2,
**dadurch gekennzeichnet,**
**dass** der Sauerstoff bei 25 °C und Normaldruck in einer Konzentration von 3 bis 200 ppm (0,002 bis 0,14 Mol-%) Sauerstoff vorliegt.

4. Verwendung nach Anspruch 3,
**dadurch gekennzeichnet,**
**dass** der Sauerstoff bei 25 °C und Normaldruck in einer Konzentration von 5 bis 100 ppm (0,004 bis 0,07 Mol-%) Sauerstoff vorliegt.

5. Verwendung nach einem der vorherigen Ansprüche,
**dadurch gekennzeichnet,**
**dass** der Sauerstoff in Form von reinem trockenen Sauerstoff und/oder trockener Luft und/oder trockener synthetischer Luft eingebracht wird.

6. Verwendung nach einem der vorherigen Ansprüche,
**dadurch gekennzeichnet,**
**dass** der Sauerstoff in einer Mischung umfassend inerte Gase verwendet wird.

7. Verwendung nach einem der vorherigen Ansprüche,
**dadurch gekennzeichnet,**
**dass** das Diisocyanat 3 bis 18 Kohlenstoffatome aufweist.

8. Verwendung nach einem der vorherigen Ansprüche,
**dadurch gekennzeichnet,**
**dass** das Diisocyanataus der Gruppe ausgewählt ist, die 1,4-Diisocyanotobutan, 2-Ethyl-1,4-diisocyanato-butan, 1,5-Diisocyanto-pentan, 2,2-Dimethyl-1,5-diisocyanato-pentan, 2-Methyl-1,5-diisocyanato-pentan (MPDI), 2-Ethyl-2-propyl-1,5-diisocyanato-pentan, 2-Ethyl-2-butyl-1,5-diisocyanato-pentan, 2-Alkoxy-1,5-diisocyanato-pentan, 1,6-Hexamethylen-diisocyanat (HDI), 2,4,4- bzw. 2,2,4-Trimethyl-1,6-hexamethylen-diisocyanat (TMDI), 1,7-Diisocyanato-heptan, 1,8-Diisocyanato-octan, 1,10-Diisocyanato-decan, 1,12-Diisocyanato-dodecan, 4,4'-Diisocyanato-dicyclohexylmethan, 2,4'-Diisocyanato-dicyclohexylmethan sowie Gemische der isomeren Diisocyanato-dicyclohexylmethane (H12MDI), 1,3-Diisocyanato-cyclohexan sowie Isomerengemische von Diisocyanato-cyclohexanen und 1-Isocyanato-3-isocyanatomethyl-3,5,5-trimethylcyclohexan (IPDI) umfasst.

9. Verwendung nach Anspruch 8,
**dadurch gekennzeichnet,**
**dass** das Diisocyanat IPDI, H₁₂MDI, TMDI, HDI und/oder MPDI umfasst.

10. Verwendung nach einem der vorherigen Ansprüche,
**dadurch gekennzeichnet,**
**dass** weiterhin Stabilisatoren ausgewählt aus der Gruppe umfassend
a) primäre Stabilisatoren und
b) sekundäre Stabilisatoren
verwendet werden.

11. Verwendung nach Anspruch 10,
**dadurch gekennzeichnet,**
**dass**
a) 1 bis 300 ppm (0,0002 bis 0,03 Mol-%) primäre Stabilisatoren oder
b) 1 bis 300 ppm (0,0002 bis 0,03 Mol-%) sekundäre Stabilisatoren verwendet werden.

12. Verwendung nach einem der vorherigen Ansprüche,
**dadurch gekennzeichnet,**
**dass** primäre Stabilisatorenverwendet werden, die aus der Gruppe umfassend 2,6-Di-tert.-butyl-4-methylphenol, 2,4,6-Tri-tert.-butylphenol, 2,2'-Methylen-bis-(4-methyl-6-tert.-butylphenol), 2,2'-Thio-bis-(4-methyl-6-t-butylphenol), 4,4'-Thio-bis-(3-methyl-6-t-butylphenol), 4,4'-Butyliden-bis-(3-methyl-6-tert.-butylphenol), 4,4'-Methyliden-bis-(2,6-di-tert.-butylphenol), 2,2'-Methyliden-bis-[4-methyl-6-(1-methylcyclohexyl)phenol], Tetrakis-[methylen-3-(3,5-di-tert.-butyl-4-hydroxyphenyl)-propionat]methan, 1,3,5-Trimethyl-2,4,6-tris(3,5-di-tert.-butyl-4-hydroxybenzyl)benzol, N,N'-Hexamethylen-bis-(3,5-di-tert.-butyl-4-hydroxy-hydrozimtsäureamid), Octadecyl-3-(3,5-di-tert.-butyl-4-hydroxyphenyl)-propionat, 1,3,5-Tris-(3,5-di-tert.-butyl-4-hydroxybenzyl)-isocyanurat, 1,1,3-Tris-(5-tert.-butyl-4-hxdroxy-2-methylphenyl)butan, 1,3,5-Tris-(3,5-di-tert.-butyl-4-hydroxybenzyl)mesitylen; Ethylenglycol-bis[3,3-bis(3'-tert.-butyl-4'-hydroxyphenyl)butyrat], 2,2'-Thiodiethyl-bis-3-(3,5-di-tert.-butyl-4-hydroxyphenyl)-propionat, 2,2'-Methylen-bis-(4-methyl-6-cyclohexylphenol), 1,6-Hexandiol-bis(3,5-di-tert.-butyl-4-hydroxyphenyl)propionat, 2,4-Bis-(n-octylthio)-6-(4-hydroxy-3,5-di-tert.-butylanilino)-1,3,5-triazin, 3,5-Di-tert.-butyl-4-hydroxybenzyl-phosphonsäurediethylester und Triethylenglykol-bis-3-(tert.-butyl-4-hydroxy-5-methylphenyl)propionat ausgewählt sind.

13. Verwendung nach einem der vorherigen Ansprüche,
**dadurch gekennzeichnet,**
**dass** als sekundäre Stabilisatoren Ester der phosphorigen Säure, wie z. B. Distearylpentaerythritdiphosphit, Tris-(nonylphenyl)-phosphit, Tris-(2,4-di-tert.-butylphenyl)phosphit, Neopentylglykoltriethylenglykoldiphosphit, Diisodecyl-pentaerythritdiphosphit, Tristearylphosphit, Trilaurylphosphit und insbesondere Triphenylphosphit, und/oder Thioether, wie z. B. 2-Methyl-1-propenyl-tert.-dodecylthioether, Cyclohexylidenmethyl-n-dodecylthioether, 3-Cyclohexen-(1)-ylidenmethyl-n-ocatadecylthioether, 3-Cyclohexen-(1)-ylidenmethyl-n-dodecylthioether, 3-Cyclohexen-(1)-ylidenmethyl-n-octylthioether, 3-Cyclohexen-(1)-ylidenmethyl-n-cyclohexylthioether, 3-Cyclohexen-(1)-ylidenmethyl-p-tolylthioether und 3-Cyclohexen-(1)-ylidenmethyl-benzylylthioether, verwendet werden.

14. Verwendung nach einem der vorherigen Ansprüche,
**dadurch gekennzeichnet,**
**dass** das Diisocyanat im Lagerbehälter durch eine sauerstoffhaltige Atmosphäre überlagert ist.

15. Verwendung nach einem der vorherigen Ansprüche,
**dadurch gekennzeichnet,**
**dass** unter Normalbedingungen 1 bis 200 ppm (0,0007 bis 0,14 Mol-%) Sauerstoff und 1 bis 200 ppm (0,0002 bis 0,02 Mol-%) eines primären Stabilisators a) zur Farbreduzierung verwendet werden.

16. Verwendung nach Anspruch 15,
**dadurch gekennzeichnet,**
**dass** unter Normalbedingungen 5 bis 100 ppm (0,0007 bis 0,07 Mol-%) Sauerstoff und 1 bis 150 ppm (0,0002 bis 0,015 Mol-%) 2,6-Di-tert.-butyl-4-methylphenol verwendet werden.

17. Verwendung nach einem der Ansprüche 1 bis 16,
**dadurch gekennzeichnet,**
**dass** das Diisocyanat aus mit Sauerstoff und/oder mit Luft stabilisiertem TMDI besteht.

18. Verwendung nach einem der Ansprüche 1 bis 16,
**dadurch gekennzeichnet,**
**dass** das Diisocyanat mit Sauerstoff und/oder mit Luft stabilisiertes TMDI und ein Trialkylphosphit umfasst.

19. Verwendung nach einem der Ansprüche 1 bis 16,
**dadurch gekennzeichnet,**
**dass** das Diisocyanat aus mit Sauerstoff und/oder mit Luft stabilisiertem H₁₂MDI besteht.

20. Verwendung nach einem der Ansprüche 1 bis 16,
**dadurch gekennzeichnet,**
**dass** das Diisocyanat aus mit Sauerstoff und/oder mit Luft stabilisiertem H₁₂MDI und 2,6-Di-tert.-butyl-4-methylphenol besteht.

21. Verwendung nach einem der Ansprüche 1 bis 16,
**dadurch gekennzeichnet,**
**dass** das Diisocyanat aus mit Sauerstoff und/oder mit Luft stabilisiertem IPDI besteht.

22. Verwendung nach einem der Ansprüche 1 bis 16,
**dadurch gekennzeichnet,**
**dass** das Diisocyanat aus mit Sauerstoff und/oder mit Luft stabilisiertem IPDI und 2,6-Di-tert.-butyl-4-methylphenol besteht.

## Claims

1. Use of oxygen for the stabilization of an aliphatic, cycloaliphatic or (cyclo)aliphatic diisocyanate and mixtures thereof, prepared by a phosgene-free process.

2. Use according to Claim 1,
**characterized in that**
the oxygen is present in a concentration of from 1 to 300 ppm (from 0.0007 to 0.21 mol%) at 25°C and standard pressure.

3. Use according to Claim 2,
**characterized in that**
the oxygen is present in a concentration of from 3 to 200 ppm (from 0.002 to 0.14 mol%) of oxygen at 25°C and standard pressure.

4. Use according to Claim 3,
**characterized in that**
the oxygen is present in a concentration of from 5 to 100 ppm (from 0.004 to 0.07 mol%) of oxygen at 25°C and standard pressure.

5. Use according to one of the preceding claims,
**characterized in that**
the oxygen is introduced in the form of pure dry oxygen and/or dry air and/or dry synthetic air.

6. Use according to one of the preceding claims,
**characterized in that**
the oxygen is used in a mixture comprising inert gases.

7. Use according to one of the preceding claims,
**characterized in that**
the diisocyanate has from 3 to 18 carbon atoms.

8. Use according to one of the preceding claims,
**characterized in that**
the diisocyanate is selected from the group encompassing 1,4-diisocyanatobutane, 2-ethyl-1,4-diisocyanatobutane, 1,5-diisocyanatopentane, 2,2-dimethyl-1,5-diisocyanatopentane, 2-methyl-1,5-diisocyanatopentane (MPDI), 2-ethyl-2-propyl-1,5-diisocyanatopentane, 2-ethyl-2-butyl-1,5-diisocyanatopentane, 2-alkoxy-1,5-diisocyanatopentane, hexamethylene 1,6-diisocyanate (HDI), 2,4,4- or 2,2,4-trimethylhexamethylene 1,6-diisocyanate (TMDI), 1,7-diisocyanatoheptane, 1,8-diisocyanatooctane, 1,10-diisocyanatodecane, 1,12-diisocyanatododecane, 4,4'-diisocyanatodicyclohexylmethane, 2,4'-diisocyanatodicyclohexylmethane and mixtures of the isomeric diisocyanatodicyclohexylmethanes (H12MDI), 1,3-diisocyanatocyclohexane, and isomer mixtures of diisocyanatocyclohexanes and 1-isocyanato-3-isocyanatomethyl-3,5,5-trimethylcyclohexane (IPDI).

9. Use according to Claim 8,
**characterized in that**
the diisocyanate comprises IPDI, H₁₂MDI, TMDI, HDI and/or MPDI.

10. Use according to one of the preceding claims,
**characterized in that**
stabilizers selected from the group encompassing
a) primary stabilizers and
b) secondary stabilizers
are furthermore used.

11. Use according to Claim 10,
**characterized in that**
a) from 1 to 300 ppm (from 0.0002 to 0.03 mol%) of primary stabilizers or
b) from 1 to 300 ppm (from 0.0002 to 0.03 mol%) of secondary stabilizers
are used.

12. Use according to one of the preceding claims, **characterized in that**
primary stabilizers selected from the group encompassing 2,6-di-tert-butyl-4-methylphenol, 2,4,6-tri-tert-butylphenol, 2,2'-methylenebis(4-methyl-6-tert-butylphenol), 2,2'-thiobis(4-methyl-6-t-butylphenol), 4,4'-thiobis(3-methyl-6-t-butylphenol), 4,4'-butylidenebis(3-methyl-6-tert-butylphenol), 4,4'-methylidenebis(2,6-di-tert-butylphenol), 2,2'-methylidenebis[4-methyl-6-(1-methylcyclohexyl)phenol], tetrakis[methylene 3-(3,5-di-tert-butyl-4-hydroxyphenyl)propionate]methane, 1,3,5-trimethyl-2,4,6-tris(3,5-di-tert-butyl-4-hydroxybenzyl)benzene, N,N'-hexamethylenebis(3,5-di-tert-butyl-4-hydroxyhydrocinnamide), octadecyl 3-(3,5-di-tert-butyl-4-hydroxyphenyl)propionate, 1,3,5-tris(3,5-di-tert-butyl-4-hydroxybenzyl) isocyanurate, 1,1,3-tris(5-tert-butyl-4-hxdroxy-2-methylphenyl)butane, 1,3,5-tris-(3,5-di-tert-butyl-4-hydroxybenzyl)mesitylene; ethylene glycol bis[3,3-bis(3'-tert-butyl-4'-hydroxyphenyl)butyrate], 2,2'-thiodiethyl bis-3-(3,5-di-tert-butyl-4-hydroxyphenyl)propionate, 2,2'-methylenebis(4-methyl-6-cyclohexylphenol), 1,6-hexanediol bis(3,5-di-tert-butyl-4-hydroxyphenyl)propionate, 2,4-bis(n-octylthio)-6-(4-hydroxy-3,5-di-tert-butyl-anilino)-1,3,5-triazine, diethyl 3,5-di-tert-butyl-4-hydroxybenzylphosphonate and triethylene glycol bis-3-(tert-butyl-4-hydroxy-5-methylphenyl)propionate are used.

13. Use according to one of the preceding claims,
**characterized in that**
the secondary stabilizers present are esters of phosphorus acid, for example distearylpentaerythritol diphosphite, tris(nonylphenyl) phosphite, tris(2,4-di-tert-butylphenyl) phosphite, neopentyl glycol triethylene glycol diphosphite, diisodecylpentaerythritol diphosphite, tristearyl phosphite, trilauryl phosphite and in particular triphenyl phosphite, and/or thioethers, for example 2-methyl-1-propenyl tert-dodecyl thioether, cyclohexylidenemethyl n-dodecyl thioether, 3-cyclohexen-1-ylidenemethyl n-octadecyl thioether, 3-cyclohexen-1-ylidenemethyl n-dodecyl thioether, 3-cyclohexen-1-ylidenemethyl n-octylthioether, 3-cyclohexen-1-ylidenemethyl n-cyclohexyl thioether, 3-cyclohexen-1-ylidenemethyl p-tolyl thioether and 3-cyclohexen-1-ylidenemethyl benzyl thioether are used.

14. Use according to one of the preceding claims,
**characterized in that**
the diisocyanate is blanketed in the storage vessel by an oxygenous atmosphere.

15. Use according to one of the preceding claims,
**characterized in that**
from 1 to 200 ppm (from 0.0007 to 0.14 mol%) of oxygen and from 1 to 200 ppm (from 0.0002 to 0.02 mol%) of a primary stabilizer a) for color reduction are used under standard conditions.

16. Use according to Claim 15,
**characterized in that**
from 5 to 100 ppm (from 0.0007 to 0.07 mol%) of oxygen and from 1 to 150 ppm (from 0.0002 to 0.015 mol%) of 2,6-di-tert-butyl-4-methylphenol are used under standard conditions.

17. Use according to one of Claims 1 to 16,
**characterized in that** the diisocyanate consists of TMDI stabilized with oxygen and/or with air.

18. Use according to one of Claims 1 to 16,
**characterized in that** the diisocyanate comprises TMDI stabilized with oxygen and/or with air and a trialkyl phosphite.

19. Use according to one of Claims 1 to 16,
**characterized in that** the diisocyanate consists of H₁₂MDI stabilized with oxygen and/or with air.

20. Use according to one of claims 1 to 16,
**characterized in that** the diisocyanate consists of H₁₂MDI and 2,6-di-tert-butyl-4-methylphenol stabilized with oxygen and/or with air.

21. Use according to one of Claims 1 to 16,
**characterized in that** the diisocyanate consists of IPDI stabilized with oxygen and/or with air.

22. Use according to one of Claims 1 to 16,
**characterized in that** the diisocyanate consists of IPDI and 2,6-di-tert-butyl-4-methylphenol stabilized with oxygen and/or with air.

## Revendications

1. Utilisation d'oxygène pour la stabilisation d'un diisocyanate aliphatique, cycloaliphatique ou (cyclo)aliphatique ainsi que de leurs mélanges, préparé(s) par un procédé exempt de prochaine.

2. Utilisation selon la revendication 1, **caractérisée en ce que** l'oxygène se trouve, à 25°C et à la pression normale, en une concentration de 1 à 300 ppm (0,0007 à 0,21% en mole).

3. Utilisation selon la revendication 2, **caractérisée en ce que** l'oxygène se trouve, à 25°C et à la pression normale, en une concentration de 3 à 200 ppm (0,002 à 0,14% en mole).

4. Utilisation selon la revendication 3, **caractérisée en ce que** l'oxygène se trouve, à 25°C et à la pression normale, en une concentration de 5 à 100 ppm (0,004 à 0,07% en mole).

5. Utilisation selon l'une quelconque des revendications précédentes, **caractérisée en ce que** l'oxygène est introduit sous forme d'oxygène pur sec et/ou d'air sec et/ou d'air synthétique sec.

6. Utilisation selon l'une quelconque des revendications précédentes, **caractérisée en ce que** l'oxygène est utilisé dans un mélange comprenant des gaz inertes.

7. Utilisation selon l'une quelconque des revendications précédentes, **caractérisée en ce que** le diisocyanate présente 3 à 18 atomes de carbone.

8. Utilisation selon l'une quelconque des revendications précédentes, **caractérisée en ce que** le diisocyanate est choisi dans le groupe comprenant le 1,4-diisocyanotobutane, le 2-éthyl-1,4-diisocyanatobutane, le 1,5-diisocyanatopentane, le 2,2-diméthyl-1,5-diisocyanatopentane, le 2-méthyl-1,5-diisocyanatopentane (MPDI), le 2-éthyl-2-propyl-1,5-diisocyanatopentane, le 2-éthyl-2-butyl-1,5-diisocyanatopentane, le 2-alcoxy-1,5-diisocyanatopentane, le diisocyanate de 1,6-hexaméthylène (HDI), le diisocyanate de 2,4,4-triméthyl-1,6-hexaméthylène ou de 2,2,4-triméthyl-1,6-hexaméthylène (TMDI), le 1,7-diisocyanatoheptane, le 1,8-diisocyanatooctane, le 1,10-diisocyanatodécane, le 1,12-diisocyanatododécane, le 4,4'-diisocyanatodicyclohexylméthane, le 2,4'-diisocyanatodicyclohexylméthane ainsi que les mélanges des diisocyanatodicyclohexylméthanes isomères (H₁₂MDI), le 1,3-diisocyanatocyclohexane ainsi que les mélanges d'isomères de diisocyanatocyclohexanes et le 1-isocyanato-3-isocyanatométhyl-3,5,5-triméthylcyclohexane (IPDI).

9. Utilisation selon la revendication 8, **caractérisée en ce que** le diisocyanate comprend de l'IPDI, du H₁₂MDI, du TMDI, de l'HDI et/ou du MPDI.

10. Utilisation selon l'une quelconque des revendications précédentes, **caractérisée en ce qu'**en outre des stabilisants choisis dans le groupe comprenant
a) des stabilisants primaires et
b) des stabilisants secondaires sont utilisés.

11. Utilisation selon la revendication 10, **caractérisée en ce que**
a) 1 à 300 ppm (0,0002 à 0,03% en mole) de stabilisants primaires ou
b) 1 à 300 ppm (0,0002 à 0,03% en mole) de stabilisants secondaires
sont utilisés.

12. Utilisation selon l'une quelconque des revendications précédentes, **caractérisée en ce que** des stabilisants primaires sont utilisés, qui sont choisis dans le groupe comprenant le 2,6-di-tert-butyl-4-méthylphénol, le 2,4,6-tri-tert-butylphénol, le 2,2'-méthylènebis-(4-méthyl-6-tert-butylphénol), le 2,2'-thio-bis-(4-méthyl-6-tert-butylphénol), le 4,4'-thio-bis-(3-méthyl-6-tert-butylphénol), le 4,4'-butylidène-bis-(3-méthyl-6-tert-butylphénol), le 4,4'-méthylidène-bis-(2,6-di-tert-butylphénol), le 2,2'-méthylidène-bis-[4-méthyl-6-(1-méthylcyclohexyl)phénol], le tétrakis-[méthylène-3-(3,5-di-tert-butyl-4-hydroxyphényl)-propionate]méthane, le 1,3,5-triméthyl-2,4,6-tris(3,5-di-tert-butyl-4-hydroxybenzyl)benzène, le N,N'-hexaméthylène-bis-(amide de l'acide 3,5-di-tert-butyl-4-hydroxyhydrocinnamique), le 3-(3,5-di-tert-butyl-4-hydroxyphényl)-propionate d'octadécyle, l'isocyanurate de 1,3,5-tris-(3,5-di-tert-butyl-4-hydroxybenzyle), le 1,1,3-tris-(5-tert-butyl-4-hxdroxy-2-méthylphényl)butane, le 1,3,5-tris-(3,5-di-tert-butyl-4-hydroxybenzyl)mésitylène, le bis[3,3-bis(3'-tert-butyl-4'-hydroxyphényl)butyrate] d'éthylèneglycol, le bis-3-(3,5-di-tert-butyl-4-hydroxyphényl)-propionate de 2,2'-thiodiéthyle, le 2,2'-méthylène-bis-(4-méthyl-6-cyclohexylphénol), le bis(3,5-di-tert-butyl-4-hydroxyphényl)propionate de 1,6-hexanediol, la 2,4-bis-(n-octylthio)-6-(4-hydroxy-3,5-di-tert-butylanilino)-1,3,5-triazine, l'ester diéthylique de l'acide 3,5-di-tert-butyl-4-hydroxybenzylphosphonique et le bis-3-(tert-butyl-4-hydroxy-5-méthylphényl)propionate de triéthylèneglycol.

13. Utilisation selon l'une quelconque des revendications précédentes, **caractérisée en ce qu'**on utilise, comme stabilisants secondaires, des esters de l'acide phosphoreux, tels que par exemple le diphosphite de distéaryl-pentaérythritol, le phosphite de tris-(nonylphényle), le phosphite de tris-(2,4-di-tert-butylphényle), le diphosphite de néopentylglycol-triéthylèneglycol, le diphosphite de diisodécyl-pentaérythritol, le phosphite de tristéaryle, le phosphite de trilauryle et en particulier le phosphite de triphényle, et/ou des thioéthers, tels que par exemple le 2-méthyl-1-propényltert-dodécylthioéther, le cyclohexylidèneméthyl-n-dodécylthioéther, le 3-cyclohexén-(1)-ylidèneméthyl-n-ocatadécylthioéther, le 3-cyclohexén-(1)-ylidèneméthyl-n-dodécylthioéther, le 3-cyclohexén-(1)-ylidèneméthyl-n-octylthioéther, le 3-cyclohexén-(1)-ylidèneméthyl-n-cyclohexylthioéther, le 3-cyclohexén-(1)-ylidèneméthyl-p-toluylthioéther et le 3-cyclohexén-(1)-ylidèneméthylbenzylylthioéther.

14. Utilisation selon l'une quelconque des revendications précédentes, **caractérisée en ce qu'**une atmosphère contenant de l'oxygène est superposée au diisocyanate dans le récipient de stockage.

15. Utilisation selon l'une quelconque des revendications précédentes, **caractérisée en ce que**, dans des conditions normales, on utilise 1 à 200 ppm (0,0007 à 0,14% en mole) d'oxygène et 1 à 200 ppm (0,0002 à 0,02% en mole) d'un stabilisant primaire a) pour la réduction de la couleur.

16. Utilisation selon la revendication 15, **caractérisée en ce que**, dans des conditions normales, on utilise 5 à 100 ppm (0,0007 à 0,07% en mole) d'oxygène et 1 à 150 ppm (0,0002 à 0,015% en mole) de 2,6-di-tert-butyl-4-méthylphénol.

17. Utilisation selon l'une quelconque des revendications 1 à 16, **caractérisée en ce que** le diisocyanate est constitué de TMDI stabilisé par de l'oxygène et/ou par de l'air.

18. Utilisation selon l'une quelconque des revendications 1 à 16, **caractérisée en ce que** le diisocyanate comprend du TMDI et un phosphite de trialkyle stabilisés par de l'oxygène et/ou par de l'air.

19. Utilisation selon l'une quelconque des revendications 1 à 16, **caractérisée en ce que** le diisocyanate est constitué de H₁₂MDI stabilisé par de l'oxygène et/ou par de l'air.

20. Utilisation selon l'une quelconque des revendications 1 à 16, **caractérisée en ce que** le diisocyanate est constitué de H₁₂MDI et de 2,6-di-tert-butyl-4-méthylphénol stabilisés par de l'oxygène et/ou par de l'air.

21. Utilisation selon l'une quelconque des revendications 1 à 16, **caractérisée en ce que** le diisocyanate est constitué d'IPDI stabilisé par de l'oxygène et/ou par de l'air.

22. Utilisation selon l'une quelconque des revendications 1 à 16, **caractérisée en ce que** le diisocyanate est constitué d'IPDI et de 2,6-di-tert-butyl-4-méthylphénol stabilisés par de l'oxygène et/ou par de l'air.
